# EUROPEAN PATENT APPLICATION

(11) **EP 2 324 818 A1**
(43) Date of publication of application: **25.05.2011**
(21) Application number: 10014176.1
(22) Date of filing: 01.11.2010
(51) Int. Cl.: A61K 8/41, A61K 8/64, A61K 8/73, A61K 8/97, A61K 8/99, A61Q 19/08

(54) **Cosmetic and health preraration with stem cells**

(30) Priority: 02.11.2009 CZ 200921885 U
(71) Applicant: Fiker, Sobeslav, 120 00 Praha 2 (CZ)
(72) Inventor: Fiker, Sobeslav, 120 00 Praha 2 (CZ)
(74) Representative: Reichel, Pavel

(57) **Abstract**

A cosmetic and health preparation with plant stem cells, to improve the functional properties of the skin, **characterised in that** it contains a combination of active substances selected from the group comprising acetyl hexapeptid-8 in a concentration of 0.0070 %, pentapeptid-3 in a concentration of 0.0030 %, acetyl glutamyl hexapeptid-1 in a concentration of 0.0025 %, sodium hyaluronate in a concentration of 0.00015 %, dimethylaminoethanol in a concentration of 0.50 %, extract of the enzyme Pseudoalteromonas in a concentration of 0.6250 %, hydrolyzed wheat protein in a concentration of 0.1430 %, hydrolyzed soy protein in a concentration of 0.0930 %, tripeptid-10 citrulin in a concentration of 0.0020 %, tripeptid-1 in a concentration of 0.0005 %, extract of *Malus domestica* plant cell culture in a concentration of 0.06 % and oligopeptid-24 in a concentration of 25 ppm.

## Description

### Field of the invention

The invention involves a cosmetic and health preparation with plant stem cells, in the form of an emulsion or serum, to improve the functional properties of the skin.

### Description of the prior art

Deterioration of the functional properties of the skin occurs due to ageing, accompanied by certain biochemical, histological and physiological changes which are more intensive during exposure of the skin to the effects of the environment. Other factors contribute to that deterioration, as for example the constant stretching of the skin under the influence of gravity, constant positional pressure on the skin on the face, for example during sleep, or repeated facial expressions caused by contraction of the facial muscles. The muscles contract when they receive acetylcholine, which is released from the vesicle in the process known as neuronal exocytosis. The aim of the present invention is to improve the functional properties of the skin, its stability and resistance to wrinkle development.

### Summary of the invention

The subject of the present invention is a cosmetic and health preparation with plant stem cells, to improve the functional properties of the skin. The basis of the invention lies in the fact that it contains a combination of active substances selected from the group comprising acetyl hexapeptid-8 in a concentration of 0.0070 %, pentapeptid-3 in a concentration of 0.0030 %, acetyl glutamyl hexapeptid-1 in a concentration of 0.0025 %, sodium hyaluronate in a concentration of 0.00015 %, dimethylaminoethanol in a concentration of 0.50 %, extract of the enzyme Pseudoalteromonas in a concentration of 0.6250 %, hydrolyzed wheat protein in a concentration of 0.1430 %, hydrolyzed soy protein in a concentration of 0.0930 %, tripeptid-10 citrulin in a concentration of 0.0020 %, tripeptid-1 in a concentration of 0.0005 %, extract of *Malus domestica* plant cell culture in a concentration of 0.06 % and oligopeptid-24 in a concentration of 25 ppm.

The cosmetic and health preparation can be in the form of an emulsion and also contains glycerin in a concentration of 3.00 %, polyakrylamide in a concentration of 0.50 %, C12-14 isoparafin in a concentration of 1.00 %, the emulsifier Laureth-7 in a concentration of 1.00 %, isopropyl myristate in a concentration of 2.00 %, dimethicone in a concentration of 2.00%, the emulsifier C12-14 Pareth-30 in a concentration of 1.60 %, glycerin stearate in a concentration of 1.2 %, cetearylalcohol in a concentration of 1.00 %, methylparaben in a concentration of 0.30 %, imidazoldinyl Urea in a concentration of 0.20 %, propylparaben in a concentration of 0.10 %, a deodorant ingredient in a concentration of 0.10 %, with the addition of 100 % water.

In another embodiment the cosmetic and health preparation can be in the form of a serum and also contains glycerin in a concentration of 11.00 %, panthenol in a concentration of 5.00 %, propylene glycol in a concentration of 3.00 %, the emulsifier polysorbate 20 in a concentration of 1.5 %, the antioxidant tocophenyl acetate in a concentration of 0.20 %, retinyl acetate in a concentration of 0.10 %, carbomer in a concentration of 0.22 %, methylparaben in a concentration of 0.30 %, propylparaben in a concentration of 0.10 %, imidazoldinyl Urea in a concentration of 0.20 %, the buffering ingredient triethanolamine up to pH 5.0 to 7.0, and a deodorant ingredient in a concentration of 0.10 %, with the addition of 100 % water.

The combination of the individual components of emulsion or serum with the above mentioned active substances improves the functional properties of the skin. Acetyl hexapeptid-8 suppresses the signals of muscle contraction and thus minimizes the emergence of wrinkles, pentapeptid-3 has a fundamental impact on reduction and relaxation of muscle contraction. Acetyl glutamyl hexapeptid-1 reduces the depth of wrinkles caused by muscle contraction, particularly around the eyes and on the forehead. Sodium hyaluronate and glycerin are hydrating ingredients. Dimethylaminoethanol stimulates synthesis of phosphatidylcholine, it is an important component of the cellular membrane and regulates viscosity, the extract of Pseudoalteromonas enzyme acts on the keratinized layer of the skin and prevents it from drying up. Hydrolyzed wheat protein and soy protein are antistatic ingredients and, with tripeptid-10 citrulin, act as a skin conditioner. Tripeptid-1 acts against mimic wrinkles and relaxes the facial muscles. The extract of *Malus domestica* plant cell culture ensures the longevity of the stem cells of the skin, oligopeptid-24 reduces wrinkles, stimulates and participates in the creation of new skin cells, improves skin tone and minimizes defects.

A mixture of polyakrylamide, C12-14 isoparafin and the emulsifier Laureth-7 acts as a film-forming ingredient, a solvent and a surfactant. Isopropylmyristate and dimethicone are smoothing ingredients, the skin conditioner C12-14 Pareth-30 and glycerinstearate are emulsifiers. The mixture of phospholipides, carbomer, triethanolamine, sodium hyaluranate, and the ingredients methylparaben, propylparaben and imidazoldinyl Urea have preservative and hydrating effects, act as tissue-building and buffering agents and regulate viscosity.

### Examples of preferred embodiments

### Example 1

The preparation contains the following combination of active ingredients: Acetyl hexapeptid-8 in a concentration of 0.0070 %, pentapeptid-3 in a concentration of 0.0030 %, acetyl glutamyl hexapeptid-1 in a concentration of 0.0025 %, sodium hyaluronate in a concentration of 0.00015 %, dimethylaminoethanol in a concentration of 0.50 %, extract of the enzyme Pseudoalteromonas in a concentration of 0.6250 %, hydrolyzed wheat protein in a concentration of 0.1430 %, hydrolyzed soy protein in a concentration of 0.0930 %, tripeptid-10 citrulin in a concentration of 0.0020 %, tripeptid-1 in a concentration of 0.0005 %, extract of *Malus domestica* plant cell culture in a concentration of 0.06 % and oligopeptid-24 in a concentration of 25 ppm.

It is in the form of an emulsion and also contains glycerin in a concentration of 3.00 %, polyakrylamide in a concentration of 0.50 %, C12-14 isoparafin in a concentration of 1.00 %, the emulsifier Laureth-7 in a concentration of 1.00 %, isopropyl myristate in a concentration of 2.00 %, dimethicone in a concentration of 2.00%, the emulsifier C 12-14 Pareth-30 in a concentration of 1.60 %, glycerin stearate in a concentration of 1.2 %, cetearylalcohol in a concentration of 1.00 %, methylparaben in a concentration of 0.30 %, imidazoldinyl Urea in a concentration of 0.20 %, propylparaben in a concentration of 0.10 %, a deodorant ingredient in a concentration of 0.10 %, with the addition of 100 % water.

### Example 2

The preparation contains the active ingredients set out in example 1. It is in the form of a serum and also contains glycerin in a concentration of 11.00 %, panthenol in a concentration of 5.00 %, propylene glycol in a concentration of 3.00 %, the emulsifier polysorbate 20 in a concentration of 1.50 %, the antioxidant tocophenyl acetate in a concentration of 0.20 %, retinyl acetate in a concentration of 0.10 %, carbomer in a concentration of 0.22 %, methylparaben in a concentration of 0.30 %, propylparaben in a concentration of 0.10 %, imidazoldinyl Urea in a concentration of 0.20 %, the buffering ingredient triethanolamine up to pH 5.0 to 7.0, and a deodorant ingredient in a concentration of 0.10 %, with the addition of 100 % water.

## Claims

1. A cosmetic and health preparation with plant stem cells, to improve the functional properties of the skin, **characterised in that** it contains a combination of active substances selected from the group comprising acetyl hexapeptid-8 in a concentration of 0.0070 %, pentapeptid-3 in a concentration of 0.0030 %, acetyl glutamyl hexapeptid-1 in a concentration of 0.0025 %, sodium hyaluronate in a concentration of 0.00015 %, dimethylaminoethanol in a concentration of 0.50 %, extract of the enzyme Pseudoalteromonas in a concentration of 0.6250 %, hydrolyzed wheat protein in a concentration of 0.1430 %, hydrolyzed soy protein in a concentration of 0.0930 %, tripeptid-10 citrulin in a concentration of 0.0020 %, tripeptid-1 in a concentration of 0.0005 %, extract of *Malus domestica* plant cell culture in a concentration of 0.06 % and oligopeptid-24 in a concentration of 25 ppm.

2. A cosmetic and health preparation according to claim 1, **characterised in that** it is in the form of an emulsion and also contains glycerin in a concentration of 3.00 %, polyakrylamide in a concentration of 0.50 %, C12-14 isoparafin in a concentration of 1.00 %, the emulsifier Laureth-7 in a concentration of 1.00 %, isopropyl myristate in a concentration of 2.00 %, dimethicone in a concentration of 2.00%, the emulsifier C 12-14 Pareth-30 in a concentration of 1.60 %, glycerin stearate in a concentration of 1.2 %, cetearylalcohol in a concentration of 1.00 %, methylparaben in a concentration of 0.30 %, imidazoldinyl Urea in a concentration of 0.20 %, propylparaben in a concentration of 0.10 %, a deodorant ingredient in a concentration of 0.10 %, with the addition of 100 % water.

3. A cosmetic and health preparation according to claim 1, **characterised in that** it is in the form of a serum and also contains glycerin in a concentration of 11.00 %, panthenol in a concentration of 5.00 %, propylene glycol in a concentration of 3.00 %, the emulsifier polysorbate 20 in a concentration of 1.5 %, the antioxidant tocophenyl acetate in a concentration of 0.20 %, retinyl acetate in a concentration of 0.10 %, carbomer in a concentration of 0.22 %, methylparaben in a concentration of 0.30 %, propylparaben in a concentration of 0.10 %, imidazoldinyl Urea in a concentration of 0.20 %, the buffering ingredient triethanolamine up to pH 5.0 to 7.0, and a deodorant ingredient in a concentration of 0.10 %, with the addition of 100 % water.
